# EUROPEAN PATENT SPECIFICATION

(11) **EP 1 643 990 B1**
(45) Date of publication and mention of the grant of the patent: **17.01.2007**
(21) Application number: 04740567.5
(22) Date of filing: 18.06.2004
(51) Int. Cl.: A61K 31/4164, A61K 47/02, A61P 17/00

(54) **METRONIDAZOLE-BASED GREEN TINTED TOPICAL PHARMACEUTICAL COMPOSITION**
GRÜN GEFÄRBTE,TOPISCHE PHARMAZEUTISCHE ZUSAMMENSETZUNG AUF DER BASIS VON METRONIDAZOL
COMPOSITION PHARMACEUTIQUE TOPIQUE DE TEINTE VERTE A BASE DE METRONIDAZOLE

(30) Priority: 18.06.2003 FR 0307354; 24.07.2003 US 489673 P
(43) Date of publication of application: 12.04.2006
(73) Proprietor: Galderma Research & Development, S.N.C., 06560 Valbonne, Sophia Antipolis (FR)
(72) Inventor: BARTHEZ, Nathalie, F-06300 Nice (FR); ORSONI, Sandrine, F-06210 Mandelieu (FR); FAUST, Jessica, South River, NJ 08882 (US); MANETTA, Vincent, Bordentown, NJ 08505 (US); FREDON, Laurent, Chemin du Camouyer, F-06330 Roquefort les pins (FR)
(74) Representative: Allab, Myriam
(86) International application number: PCT/EP2004/007207
(87) International publication number: WO 2004/112780

(56) References cited:
- WO-A-89/06537
- US-A- 5 593 680
- US-A- 5 660 839
- US-A1- 2002 192 272

## Description

The present invention relates to a tinted topical pharmaceutical composition for human use comprising, in a pharmaceutically acceptable vehicle, metronidazole and at least one dye, and also to the use of this composition for preparing a medicinal product for treating dermatological complaints, such as rosacea.

Rosacea is a pathology that affects close to one in twenty Americans and one in fifteen British people. Although it mainly affects Western and Northern European adults in equal proportions, rosacea also affects Asiatic, African and Hispanic populations. It appears that rosacea affects women more frequently, but men more severely.

The literature suggests that rosacea is an abnormal vascular response of genetic origin leading to vasodilation, the mechanism of which is not really elucidated at the present time. No rosacea-related gene is known to date. The various clinical signs are vasodilation-related symptoms, namely diffuse redness, erythema, telangiectasia, or rhinophyma, but also signs that may be linked to proliferation of the demodex folliculorum and to inflammation, namely papules and pustules.

The common therapies for rosacea are limited to controlling the symptoms; they slow down, but do not stop, the progression of the disease. Individuals suffering from rosacea are thus obliged to adopt a lifestyle linked to their pathology.

Among the existing treatments, topical treatments are essentially based on metronidazole (see US 5 593 6880 A and US 5 660 839 A), sulphur derivatives or sodium sulphacetamide.

Metronidazole is the first of many nitroimidazole antimicrobial agents, which was introduced into human and veterinary medicine in 1959. As a result of its antiprotozolan and antibacterial activity, metronidazole was introduced for the treatment of trichomonas, but its therapeutic indications became diversified and broadened as the years passed, to make it one of the ten most widely distributed (in quantity) medicinal products worldwide.

It was in 1975 that beneficial changes on the skin were accidentally observed in the case of patients suffering from rosacea who were treated with metronidazole for other reasons. Thus, two British doctors reported the following year that metronidazole at a rate of 200 mg twice a day had therapeutic effects on the papules and pustules of rosacea, but was ineffective on erythema. It was not until ten years later that metronidazole was recognized as being an effective treatment against rosacea.

The first metronidazole-based topical formulation for rosacea was marketed by the Applicant in 1991.

The products Métrogel^{®}, Rosex^{®} gel, MetroLotion^{®} or Métrocreme^{®} marketed by the Applicant, for treating rosacea, will be noted in particular at the present time. Metronidazole effectively improves the inflammatory lesions and is also found to be effective against the papules and pustules, but has no action on the clinical signs such as erythema, telangiectasia or redness.

Now, there are numerous patients affected by this pathology who suffer from these clinical signs, which have impacts on their social and professional life, as demonstrated by certain statistical studies. Specifically, out of 700 patients suffering from rosacea, more than 60% indicate that the clinical signs substantially affected their professional life, going as far as to hinder their professional recruitment and prevent a normal social life.

There is thus a real need not only to treat the pathology, but also to minimize these clinical signs, which are highly incapacitating for the patient.

Now, the Applicant has just developed a novel topical pharmaceutical composition that combines metronidazole with at least one dye, for the preparation of a medicinal product for simultaneously treating the pathology and the associated clinical signs described above.

This composition also has the advantage of being easy for the patient to use (only one product) and of being compatible with the active properties of metronidazole. The association between the active agent and the dye must, specifically, preserve the stability of the active agent and of the composition, maintain good bioavailability of the active principle, and also good tolerance, similar to the pharmaceutical product in the absence of dye.

By the present invention, the Applicant is thus proposing a physically and chemically stable composition for combining within a single product the pharmaceutical active agent and at least one dye in order to mask the redness, while at the same time controlling any possible interactions, and maintaining a bioavailability of metronidazole that is equivalent to that of dye-free products.

The invention thus relates to a physically and chemically stable tinted pharmaceutical composition for masking redness, comprising metronidazole and at least one dye.

According to the invention, the expression "tinted pharmaceutical composition for masking redness" means a green tinted composition. The composition according to the invention thus comprises one or more dyes so as to give the composition the desired green colour. The concept of the green colour is chosen according to the colour opposition principle symbolized by the chromatic circle. The intensity of a colour may be reduced by addition of its opposite colour on the chromatic circle on which all colours are represented, each having its opposite. In all the artistic fields, the sole way of neutralizing a colour spot is to mix it with its opposite. Since green is opposite red on the chromatic circle, a green colour is chosen to colour the composition according to the invention so as to neutralize the diffuse redness of the skin of patients suffering from rosacea.

Dyes are incorporated into the composition in order to give the composition according to the invention the desired green colour.

The term "dye" means the generic term used by those skilled in the art, defining a substance that gives a colour to a medium (article by Gisbert Otterstätter: "Coloring Cosmetics, Cosmetics & Toiletries Magazine; Vol 111, March 1996, pages 25-33), such as:
- dyes defined as being soluble in the medium to be coloured; they are often liposoluble or water-soluble,
- pigments or colour lakes, which are generally insoluble in the medium to be coloured, the lakes being obtained by precipitating water-soluble dyes with a water-insoluble salt such as an aluminium hydroxide,
- water-dispersible pigments, which, by addition of a solvent, give water-stable dispersions that allow their use as dyes,
alone or as a mixture.

As examples of dyes that may be used according to the invention, mention may be made of pigments such as brown, yellow or red iron oxides or hydroxides, chromium oxides or hydroxides, titanium oxides or hydroxides, mixtures of blue and yellow dyes such as the lakes referenced FD&C Blue No. 1 aluminium lake (E133), FD&C Blue No. 2 aluminium lake (E132), FD&C Yellow No. 5 aluminium lake (E102) or FD&C Yellow No. 6 aluminium lake (E110).

The dyes according to the invention may be used alone or as mixtures to give the composition according to the invention the desired green colour.

According to one preferred mode of the invention, the composition also comprises titanium oxides along with other dyes, for their covering property.

The dyes chosen according to the invention will also need to be able to be registered with drug agencies in order to be able to be used in the pharmaceutical composition according to the invention. The article by David R. Schoneker ("Coloring agents for use in pharmaceuticals", *Encyclopedia of Pharmaceutical Technology,* 2002, pages 509-530) is a review of various established rules regarding dyes in order to classify them according to their use. A division into three categories is described therein; food, drug and cosmetic dyes, classified as FD&C, drug and cosmetic (D&C) dyes, and dyes for externally applied drugs and for cosmetics (external D&C). The "Handbook of Pharmaceutical Excipients" (The Pharmaceutical Press, Third Edition, pages 146-153) may also be cited as a reference book.

A composition according to the invention preferably contains a total amount of dyes ranging from 0.001% to 10% by weight of the composition, it being understood that, preferably, the total amount of dyes cannot exceed 1% by weight of the composition. Each dye is used in proportions ranging from 0.0001% to 1% by weight of the composition.

The dye mixtures preferably used for the composition according to the invention are the following:
- FD&C Blue No. 2 aluminium lake + yellow iron oxide + titanium dioxide
- chromium hydroxide + yellow iron oxide + titanium dioxide
- FD&C Blue No. 1 aluminium lake + yellow iron oxide + titanium dioxide
- chromium hydroxide + titanium dioxide

The active agent incorporated into the composition according to the invention is metronidazole. The term "metronidazole" especially means 1-(2-hydroxyethyl)-2-methyl-5-nitroimidazole, but also the analogues and derivatives thereof.

The composition according to the invention preferably contains from 0.001% to 10% by weight of metronidazole relative to the total weight of the composition. More preferably, the composition according to the invention contains from 0.05% to 1% by weight of metronidazole relative to the total weight of the composition.

The composition according to the invention is a composition with good physical and chemical stability.

According to the invention, the term "physical stability" means a composition that is stable at any temperature conventionally used by a person skilled in the art to predict the stability of a tinted pharmaceutical topical product. The composition must be stable at room temperature, at 45°C and at 4°C for three months. During this time, it should not show any change in macroscopic appearance, such as a change in colour or leaching of pigment, or in the microscopic appearance, such as recrystallization of the active agent.

According to the invention, the term "chemical stability" means no change in the concentration of the active agent over time caused by the presence of pigments. The addition of pigments to the formulation should not modify the efficacy of the active agent and should not cause any chemical degradation thereof over time.

As a result of its composition, the pharmaceutical composition according to the invention is intended for treating the skin and may be in liquid, pasty or solid form, and more particularly in the form of ointments, creams, milks, salves, powders, impregnated pads, syndets, wipes, solutions, gels, sprays, mousses, suspensions, lotions, sticks, shampoos or washing bases.

The composition may also be in the form of suspensions of lipid or polymer vesicles, nanospheres or microspheres or polymer patches and hydrogels allowing a controlled release. This composition for topical application may be in anhydrous form, in aqueous form or in the form of an emulsion.

In one preferred variant of the invention, the pharmaceutical composition according to the invention is in the form of a gel, a cream or a lotion.

The composition according to the invention is more preferably in the form of a cream.

The pharmaceutical composition according to the invention may also contain inert additives or combinations of these additives, such as
- wetting agents;
- flavour enhancers;
- preserving agents;
- stabilizers;
- humidity regulators;
- pH regulators;
- osmotic pressure modifiers;
- emulsifiers;
- UV-A and UV-B screening agents; and
- antioxidants.

Needless to say, a person skilled in the art will take care to select the optional compound(s) to be added to these compositions and the respective amounts thereof, such that the advantageous properties intrinsically associated with the present invention are not, or are not substantially, adversely affected by the envisaged addition.

The invention also relates to the procedure for the composition according to the invention. During the formulation of a tinted composition, the most important step is the step of incorporating the dyes. The dyes are preferably incorporated into the oily phase by dispersion with vigorous stirring.

The term "vigorous stirring" especially means a minimum speed of 1000 rpm using a Rayneri blender or Ultra-Turrax stirring; the stirring will preferably be performed by Ultra-Turrax stirring at 8000 rpm for at least 20 minutes.

The oily phase containing the dyes is then added to the aqueous phase and the phases are emulsified for 10 minutes at a speed of between 1000 and 1500 rpm using a Rayneri blender at high temperature (between 60 and 85°C).

The temperature and stirring speed are then reduced so as to obtain the desired viscosity according to the type of formulation produced. The viscosities of the various formulations in the cosmetic or pharmaceutical field (lotion, milk, cream, salve, etc.) are well known to those skilled in the art.

This procedure thus makes it possible to prevent any sedimentation of the pigments in the formulation from being observed during centrifugation controls at 10 000 rpm for 15 minutes, or at 3000 rpm for 30 minutes.

The term "moderate stirring" means stirring obtained using a machine of Rayneri type at a speed of between 600 and 1000 rpm, and "gentle stirring" means stirring obtained using a machine of Rayneri type at a speed of less than 600 rpm.

The invention also relates to the use of the composition according to the invention for the manufacture of a medicinal product for treating dermatological complaints.

The term "dermatological complaints" more particularly means rosacea, common acne, seborrhoeic dermatitis, peroral dermatitis, acneiform eruptions, transient acantholytic dermatitis and acne miliaris necrotica.

The composition according to the invention is particularly suitable for treating rosacea.

Various formulations of green tinted compositions comprising metronidazole and procedures for obtaining these compositions will now be given, by way of illustration.

### EXAMPLE 1: Cream formulation

| Phases | Constituents | % (w/w) |
|---|---|---|
| A1 | purified water | qs. 100% |
| A1 | glycerol | 4.00 |
| A1 | sorbitol | 5.00 |
| A2 | metronidazole | 0.75 |
| | | |
| B1 | isopropyl palmitate | 2.00 |
| B1 | self-emulsifying wax | 12.50 |
| B2 | yellow iron oxide | 0.0045 |
| B2 | green chromium oxide | 0,020 |
| B3 | titanium dioxide | 0.50 |
| | | |
| C | benzyl alcohol | 2.20 |
| | | |
| D | 90% lactic acid | qs pH 5 |
| | | |

### PREPARATION OF THE PHASES - PROCEDURE

### FATTY PHASE B

The self-emulsifying wax is dissolved in the isopropyl palmitate (B1) at 75°C with stirring (gentle Rayneri stirring) to produce a clear homogeneous phase.

The yellow pigment is then introduced with Rayneri stirring, followed by the green pigment (B2) and the titanium dioxide (B3). The mixture is dispersed for 30 minutes (moderate Rayneri stirring) to produce a homogeneous green phase.

### AQUEOUS PHASE A

The glycerol and the sorbitol (A1) are homogenized in purified water at 75°C with stirring (gentle Rayneri stirring).

The metronidazole (A2) is then introduced and correct dissolution thereof is checked.

The mixture is emulsified at 75°C by introducing phase B (B1 + B2) into phase A (moderate Rayneri stirring), and this temperature is maintained for 10 minutes.

The mixture is cooled to 45°C. The benzyl alcohol is introduced with stirring (moderate Rayneri stirring) and the whole is homogenized.

The resulting mixture is cooled to 25°C with stirring, and the pH is measured, if necessary with qs pH 5 adjustment. This procedure gives a pale green cream.

### EXAMPLE 2: Lotion formulation

| Phases | Constituents | % (w/w) |
|---|---|---|
| A1 | purified water | qs 100% |
| A1 | glycerol | 7.00 |
| A2 | Carbomer 981 | 0.15 |
| A3 | PEG 400 | 2.00 |
| A4 | Steareth 21 | 3.00 |
| A5 | metronidazole | 0.75 |
| | | |
| B1 | yellow iron oxide | 0.0045 |
| B1 | green chromium oxide | 0.02 |
| B1 | titanium dioxide | 0.50 |
| B1 | mineral oil | 6.00 |
| B2 | glyceryl and PEG 100 stearate | 3.00 |
| B2 | stearyl alcohol | 2.00 |
| | | |
| C | benzyl alcohol | 1.30 |
| | | |
| D | purified water | 2,00 |
| D | potassium sorbate | 0.20 |
| | | |
| E | cyclomethicone 5 | 4.00 |
| | | |
| F | 10% sodium hydroxide solution | Qs pH 5 |
| F | 1 % lactic acid (90%) solution | Qs pH 5 |

### PREPARATION OF THE PHASES - PROCEDURE

### FATTY PHASE B

The yellow and green pigments are dispersed in the mineral oil with stirring (moderate Rayneri stirring) for 15 minutes at 80°C. The titanium dioxide is then introduced, and the mixture is homogenized for 15 minutes.

The temperature is maintained at 80°C and the glyceryl and PEG 100 stearate and the stearyl alcohol (B2) are then introduced, and this mixture is homogenized until the waxes have fully dissolved, to give a green homogeneous phase.

### PHASE D

The potassium sorbate is dissolved in the purified water.

### AQUEOUS PHASE A

The Carbomer 981 is dispersed in the purifed water and the glycerol (moderate Rayneri speed) at 80°C.

The PEG 400 (A3) and the steareth 21 (A4) are introduced and the mixture is homogenized for 5 minutes. The metronidazole (A5) is then introduced and its correct dissolution is checked.

The mixture is emulsified at 80°C by introducing fatty phase B into aqueous phase A with stirring (moderate Rayneri stirring). The mixture is homogenized for 10 minutes.

The resulting mixture is cooled.

Phases C, D and E are introduced at 40°C.

The resulting mixture is cooled to 25°C with stirring and the pH is measured and adjusted, if necessary, with 10% sodium hydroxide solution or 1% lactic acid solution, qs pH 5.

The procedure gives a pale green gelled fluid.

### EXAMPLE 3: Cream formulation

| Phases | Constituents | % (W/W) |
|---|---|---|
| A1 | purified water | qs 100% |
| A1 | glycerol | 4.00 |
| A1 | sorbitol | 5.00 |
| A2 | metronidazole | 0.75 |
| | | |
| B1 | isopropyl palmitate | 2.00 |
| B1 | self-emulsifying wax | 12.50 |
| B2 | yellow iron oxide | 0.0095 |
| B2 | FD&C Blue aluminium lake | 0.0068 |
| B3 | titanium dioxide | 0.50 |
| | | |
| C | benzyl alcohol | 2.20 |
| | | |
| D | 90% lactic acid qs p H5 | q s pH 5 |
| | | |

### Aqueous phase

The glycerol, sorbitol, benzyl alcohol and purified water are weighed out in a beaker. The metronidazole is then introduced.

The phase is heated to 75°C and the active agent is then dissolved with moderate stirring for 20 minutes.

### Fatty phase

The self-emulsifying wax and the isopropyl palmitate are weighed out in a beaker. The phase is heated to 75°C and homogenized by Ultra-Turrax stirring. The pigments weighed out together and the titanium dioxide are then introduced with Ultra-Turrax stirring at 8000 rpm for at least 20 minutes.

### Emulsification and cooling

The mixture is emulsified at 75°C for 10 minutes with moderate Rayneri stirring while introducing the fatty phase into the aqueous phase.

The resulting mixture is cooled with gentle stirring.

A thick homogeneous green cream is obtained.

### EXAMPLE 4: Cream formulation

| Phases | Constituents | % (w/w) |
|---|---|---|
| A1 | purified water | qs 100% |
| A1 | glycerol | 4.00 |
| A1 | sorbitol | 5.00 |
| A2 | metronidazole | 0.75 |
| | | |
| B1 | isopropyl palmitate | 2.00 |
| B1 | self-emulsifying wax | 12.50 |
| B2 | yellow iron oxide | 0.0045 |
| B2 | green chromium oxide | 0.020 |
| B3 | titanium dioxide | 0.50 |
| | | |
| C | benzyl alcohol | 2.20 |
| | | |
| D | 90% lactic acid qs pH 5 | qs pH 5 |
| | | |

The procedure is the same as that of Example 3.

### EXAMPLE 5: Cream formulation

| Phases | Constituents | % (w/w) |
|---|---|---|
| A1 | purified water | qs 100% |
| A1 | glycerol | 4.00 |
| A1 | sorbitol | 5.00 |
| A2 | metronidazole | 0.75 |
| | | |
| B1 | isopropyl palmitate | 2.00 |
| B1 | self-emulsifying wax | 12.50 |
| B2 | yellow iron oxide | 0.019 |
| B2 | FD&C Blue aluminium lake | 0.0136 |
| B3 | titanium dioxide | 1.00 |
| | | |
| C | benzyl alcohol | 2.20 |
| | | |
| D | 90% lactic acid qs pH5 | qs pH 5 |
| | | |

The procedure is the same as that of Example 3.

### EXAMPLE 6: Results of physical and chemical stability of the compositions according to examples 1, 3 and 4 above

The physical stability of the formulations is measured by a macroscopic and microscopic observation of the formulation at room temperature, at 4°C and at 45°C after 4, 8 and 12 weeks.

At room temperature, the macroscopic observation makes it possible to ensure the physical integrity of the products and the microscopic observation makes it possible to check that there is no recrystallization of the dissolved active agent and no significant change in the size of the emulsion globules.

At 4°C, the microscopic observation checks that the dissolved active agent has not recrystallized.

At 45°C, the macroscopic observation checks the integrity of the finished product.

The formulations described in Examples 1, 3 and 4 were tested. No recrystallization of the product and no phase separation over time, either at room temperature, at 4°C or at 45°C, were observed.

### a) Measurement of the pH

| composition of example | time in months | | | |
|---|---|---|---|---|
| | 0 | 1 | 2 | 3 |
| 1 | 5.1 | 5.1 | 5.1 | 5.1 |
| 3 | 5.2 | 5.4 | 5.3 | 5.2 |
| 4 | 5.2 | 5.2 | 5.0 | 5.0 |

### b) Measurement of the macroscopic parameters at a temperature of 4°C

| | time in months | | | |
|---|---|---|---|---|
| macroscopic appearance* | 0 | 1 | 2 | 3 |
| Ex.1 | homogeneous green formulation, no recrystallization of active agent | in accordance with T0 | in accordance with T0 | in accordance with T0 |
| Ex.3 | homogeneous green formulation, no recrystallization of active agent. | in accordance with T0 | in accordance with T0 | in accordance with T0 |
| Ex.4 | homogeneous green formulation, no recrystallization of active agent | in accordance with T0 | in accordance with T0 | in accordance with T0 |

| | | | | |
|---|---|---|---|---|
| *Observation of the homogeneity of the formulation and checking of the absence of recrystallization of the active agent. | | | | |

### c) Measurement of the macroscopic parameters at a temperature of 45°C

| | time in months | | | |
|---|---|---|---|---|
| macroscopic appearance* | 0 | 1 | 2 | 3 |
| Ex. 1 | homogeneous green formulation, no phase separation | in accordance with T0 | in accordance with T0 | in accordance with T0 |
| Ex.3 | homogeneous green formulation, no phase separation | in accordance with T0 | in accordance with T0 | in accordance with T0 |
| Ex.4 | homogeneous green formulation, no phase separation | in accordance with T0 | in accordance with T0 | in accordance with T0 |

| | | | | |
|---|---|---|---|---|
| *Observation of the homogeneity of the formulation, phase separation, colour | | | | |

### d) Measurement of the flow threshold

A VT 500 Haake rheometer with an SVDIN measuring spindle is used.

The rheograms were produced at 25°C and at a shear rate of 4 s⁻¹ (y), and by measuring the shear stress.

The term "flow threshold" (τ0 expressed in pascals) means the force (minimum shear stress) required to overcome the Van der Waals cohesion forces and to bring about flow. The flow threshold is likened to the value found at a shear rate of 4 s⁻¹.

These measurements are taken at T0 and after 1, 2 and 3 months on Examples 3 and 4.

| | time in months | | | |
|---|---|---|---|---|
| | 0 | 1 | 2 | 3 |
| Ex.3 | nr | 52 | 73* | 69* |
| Ex.4 | 53 | 53 | 48 | 50 |

| | | | | |
|---|---|---|---|---|
| *Use of a Haake VT 550 rheometer inducing a slight increase in the characteristic viscosity values. | | | | |

The results obtained do not show any significant variation in the flow threshold; the viscosity of the composition according to the invention is thus stable over time.

### e) Chemical stability: Measurement of the percentage of metronidazole over time at a temperature of 25°C and a relative humidity of 60% (HPLC assay of the active agent)

| | time in months | | | |
|---|---|---|---|---|
| % metronidazole | 0 | 1 | 2 | 3 |
| Ex.1 | 99.9 | 99.8 | 100.7 | 99.3 |

### f) Chemical stability: Measurement of the percentage of metronidazole over time at a temperature of 40°C and a relative humidity of 75% (HPLC assay of the active agent)

| | time in months | | | |
|---|---|---|---|---|
| % metronidazole | 0 | 1 | 2 | 3 |
| Ex.1 | 99.9 | 100.4% | 100.6 | 99.1 |

### EXAMPLE 7: Results of physical and chemical stability of the composition according to example 5

As described above, the physical stability of the formulations is measured by macroscopic and microscopic observation of the formulation at room temperature, at 4°C and at 45°C, after 4, 8 and 12 weeks.

At room temperature, the macroscopic observation makes it possible to ensure the physical integrity of the products and the microscopic observation makes it possible to check that there is no recrystallization of the dissolved active agent and no significant change in the size of the emulsion globules.

At 4°C, the microscopic observation checks that the dissolved active agent has not recrystallized.

At 45°C, the macroscopic observation checks the integrity of the finished product.

### a) Measurement of the various parameters at a temperature of 25°C and a relative humidity of 60%

| | time in months | | | |
|---|---|---|---|---|
| | 0 | 1 | 2 | 3 |
| macroscopic appearance | homogenous bright green formulation | in accordance with T0 | | |
| pH | 5.0 | 5.0 | | |
| centrifugation at 10 000 rpm for 15 minutes | no sedimentation or phase separation | in accordance with T0 | | |
| centrifugation at 3000 rpm for 30 minutes | no sedimentation or phase separation | in accordance with T0 | | |
| % metronidazole | 100.1 | 102.0 | | |

### b) Measurement of the various parameters at a temperature of 40°C and a relative humidity of 75%.

| | time in months | | | |
|---|---|---|---|---|
| | 0 (at RT) | 1 | 2 | 3 |
| macroscopic appearance | homogeneous bright green formulation | in accordance with T0 | | |
| pH | 5.0 | 5.0 | | |
| centrifugation at 10 000 rpm for 15 minutes | no sedimentation or phase separation | in accordance with T0 | | |
| centrifugation at 3000 rpm for 30 minutes | no sedimentation or phase separation | in accordance with T0 | | |
| % metronidazole | 100.1 | 102.0 | | |

These various results demonstrate good physical stability of the compositions and good chemical stability of the active agent in the various compositions according to the invention, over time.

### Example 8: Study of release-penetration of the active agent in the compositions according to the invention

The object of the study is to compare the release/penetration into human skin (without occlusion) of metronidazole:
- formulated at 0.75% in the two compositions according to the invention of Examples 3 and 4 above; and
- with a commercial reference product (Rosex^{®} Cream 0.75%).

The absorption studies were performed using excised human skin, with a diffusion cell device. Three samples of human skin obtained from three women (49 to 58 years old) were used. 10 mg of each composition (corresponding to 75 µg of metronidazole) are applied to an area of 1 cm². The concentrations of metronidazole in the receiving fluid and in the skin are measured over time by an HPLC method with UV detection (10 ng.ml⁻¹ quantification limit).

The results show that, irrespective of the test composition, the pharmaceutical active agent is distributed in the skin (epidermis including the stratum corneum and the dermis) and in the collected fractions. For each of the formulations, the metronidazole diffuses continuously through the skin to the receiving fluid during the experiment. At the end of the application period, the cumulative amounts of metronidazole found in the receiving fluid are 1.4 to 1.6 times lower than those found in the total skin.

The total amounts of metronidazole which penetrated (stratum corneum + epidermis + dermis + receiving fluid) are:
Rosex cream 0.75%: 11.31 ± 1.49 µg (14.8% of the applied dose
Composition according to Example 3: 10.18 ± 0.93 µg (14.1% of the applied dose)
Composition according to Example 4: 10.54 ± 0.85 µg (13.8% of the applied dose)

In conclusion, no significant difference was observed between the two creams according to the invention and the commercial reference product in terms of skin penetration, absorbed dose and total penetrated dose, thus demonstrating the good release and penetration of the metronidazole in the compositions according to the invention.

### Example 9: Study of the covering behaviour of the compositions according to the invention

The main object of the test is to evaluate and compare the covering power of the compositions with respect to cutaneous erythema.

The evaluation of the covering power takes place via a sensory evaluation performed with panellists trained to evaluate this descriptor.

The secondary object is to evaluate the capacity of the compositions to restore the most natural possible complexion, i.e. to cancel out skin redness as efficiently as possible, by means of their green pigments.

The objective evaluation of the skin redness is made by chromometric measurement (parameter a) performed before and after application of each product. The colorimetric measurement of the skin is performed using a Minolta CR300 chromameter equipped with an 8 mm head. The chromameter converts colours located in the range of human perception and a digital code composed of three parameters: L represents the lightness (from dark to pale), a represents the range from greens to reds, b represents the range from blues to yellows. In the evaluation of the skin redness, the parameter is of prime importance and is the only one evaluated.

The evaluation of the complexion of the face is made after applying the product using a linear scale ranging from 0 to 15, i.e. from pink-beige to pale green passing through beige.

Another object is also to evaluate the tolerance of the compositions by recording the adverse effects.

The following compositions were tested:
- a comparator, Avène rich emulsion, commercial product chosen on account of its known covering power;
- the composition according to Example 3,
- the composition according to Example 4;
- the composition according to Example 5;
- the vehicle for the compositions.

Each product is tested on the whole face of each volunteer during a sensory evaluation session. At T0, the face of the volunteers is observed and the parameter a is measured. The product is applied in a standardized manner to the whole face of the volunteers. At T10 minutes, a new observation of the face of the volunteers is performed. A new measurement of the parameter a is taken.

### Results:

The data make it possible to demonstrate a significant difference between the various test products regarding the "covering power" descriptor (p < 0.001). The compositions of Examples 3 and 4 according to the invention have a higher covering power than the vehicle and are at least as covering as the comparator.

The composition of Example 5 has a significantly higher covering power than any of the other products.

It is seen from the results that the parameter a is accentuated after applying the vehicle, whereas it decreases after applying the various test compositions, and does so in a markedly significant manner after applying the composition according to Example 5.

The results of the measurement of the "skin complexion" parameter make it possible to demonstrate that the compositions of Examples 3 and 4 give a skin complexion that is closer to beige than that obtained after applying the vehicle. The composition according to Example 5 is the one that restores a skin complexion that is significantly the closest to beige and differs for this descriptor from all the other products.

In terms of tolerance, all the test products were very well tolerated.

## Claims

1. Topical pharmaceutical composition comprising, in a pharmaceutically acceptable vehicle:
a) metronidazole,
b) at least one dye,
**characterized in that** the composition has a green colour.

2. Composition according to Claim 1,
**characterized in that** it comprises between 0.05% and 1% of metronidazole relative to the total weight of the composition.

3. Composition according to either of Claims 1 and 2, **characterized in that** the composition is a cream.

4. Composition according to one of Claims 1 to 3, **characterized in that** the dyes are chosen from pigments and lakes, taken alone or as a mixture.

5. Composition according to one of Claims 1 to 4, **characterized in that** the dyes are chosen from the following mixtures:
a) FD&C Blue No. 2 aluminium lake + yellow iron oxide + titanium dioxide
b) chromium hydroxide + yellow iron oxide + titanium dioxide
c) FD&C Blue No. 1 aluminium lake + yellow iron oxide + titanium dioxide
d) chromium hydroxide + titanium dioxide.

6. Composition according to one of Claims 1 to 5, **characterized in that** the total amount of dyes does not exceed 1% by weight of the composition.

7. Composition according to one of Claims 1 to 6, **characterized in that** it comprises:
| | |
|---|---|
| a) | Purified water |
| b) | glycerol |
| c) | sorbitol |
| d) | metronidazole |
| e) | isopropyl palmitate |
| f) | self-emulsifying wax |
| g) | yellow iron oxide |
| h) | blue dye: FD C Blue aluminium lake |
| i) | titanium dioxide |
| j) | benzyl alcohol |
| k) | 90% lactic acid qs pH5 |

8. Use of the composition according to one of the preceding claims, for the manufacture of a medicinal product for treating dermatological complaints, such as rosacea, common acne, seborrhoeic dermatitis, peroral dermatitis, acneiform eruptions, transient acantholytic dermatitis and acne miliaris necrotica.

9. Use of the composition according to Claim 8, **characterized in that** the dermatological complaint is rosacea.

10. Procedure for manufacturing the composition according to one of Claims 1 to 8, **characterized in that** it consists in:
a) incorporating the dyes into the oily phase by dispersion with vigorous stirring, preferably using an Ultra-Turrax blender, preferentially an Ultra-Turrax blender at a speed of 8000 rpm;
b) adding the oily phase containing the dyes to the aqueous phase;
c) emulsifying the phases for 10 minutes with vigorous stirring;
d) reducing the stirring speed in order to obtain the viscosity desired for a given formulation.

## Patentansprüche

1. Topische pharmazeutische Zusammensetzung, die in einem pharmazeutisch akzeptablen Vehikel enthält:
a) Metronidazol,
b) mindestens einen Farbstoff,
**dadurch gekennzeichnet, dass** die Zusammensetzung eine grüne Farbe hat.

2. Zusammensetzung nach Anspruch 1, **dadurch gekennzeichnet, dass** sie 0,05 bis 1 % Metronidazol enthält, bezogen auf das Gesamtgewicht der Zusammensetzung.

3. Zusammensetzung nach einem der Ansprüche 1 und 2, **dadurch gekennzeichnet, dass** die Zusammensetzung eine Creme ist.

4. Zusammensetzung nach einem der Ansprüche 1 bis 3, **dadurch gekennzeichnet, dass** die Farbstoffe unter Pigmenten und Farblacken, einzeln oder im Gemisch, ausgewählt werden.

5. Zusammensetzung nach einem der Ansprüche 1 bis 4, **dadurch gekennzeichnet, dass** die Farbstoffe unter den folgenden Gemischen ausgewählt werden:
a) FD&C Blue No. 2 Aluminiumfarblack + gelbes Eisenoxid + Titandioxid,
b) Chromhydroxid + gelbes Eisenoxid + Titandioxid,
c) FD&C Blue No. 1 Aluminiumfarblack + gelbes Eisenoxid + Titandioxid,
d) Chromhydroxid + Titandioxid.

6. Zusammensetzung nach einem der Ansprüche 1 bis 5, **dadurch gekennzeichnet, dass** die Gesamtmenge der Farbstoffe 1 Gew.-% der Zusammensetzung nicht übersteigt.

7. Zusammensetzung nach einem der Ansprüche 1 bis 6, **dadurch gekennzeichnet, dass** sie enthält:
a) gereinigtes Wasser,
b) Glycerin,
c) Sorbit,
d) Metronidazol,
e) Isopropylpalmitat,
f) selbstemulgierendes Wachs,
g) gelbes Eisenoxid,
h) blauen Farbstoff: FD&C Blue Aluminiumfarblack,
i) Titandioxid,
j) Benzylalkohol,
k) 90 % Milchsäure qs pH 5

8. Verwendung der Zusammensetzung nach einem der vorhergehenden Ansprüche zur Herstellung eines medizinischen Produkts zur Behandlung dermatologischer Beschwerden, wie Rosacea, Acne vulgaris, seborrhoisches Exzem, periorale Dermatitis, akneförmige Ausschläge, vorübergehende acantholytaische Dermatitis und Acne miliaris necrotica.

9. Verwendung der Zusammensetzung nach Anspruch 8, **dadurch gekennzeichnet, dass** es sich bei den dermatologischen Beschwerden um Rosacea handelt.

10. Verfahren zur Herstellung der Zusammensetzung nach einem der Ansprüche 1 bis 8, **dadurch gekennzeichnet, dass** es darin besteht:
a) die Farbstoffe durch Dispergieren unter kräftigem Rühren, vorzugsweise unter Verwendung eines Ultra-Turrax-Mischers, vorzugsweise eines Ultra-Turrax-Mischers mit einer Geschwindigkeit von 8000 U/min, in die Ölphase einzubringen,
b) die Ölphase, die die Farbstoffe enthält, in die wässrige Phase einzubringen;
c) die Phasen 10 min unter kräftigem Rühren zu emulgieren;
d) die Rührgeschwindigkeit zu senken, um für eine gegebene Formulierung die gewünschte Viskosität zu erhalten.

## Revendications

1. Composition pharmaceutique topique comprenant dans un véhicule pharmaceutiquement acceptable :
a) du métronidazole,
b) au moins un agent colorant,
**caractérisée en ce que** la composition a une couleur verte.

2. Composition selon la revendication 1 **caractérisée en ce qu'**elle comprend entre 0.05 et 1% de métronidazole par rapport au poids total de la composition.

3. Composition selon l'une des revendications 1 à 2, **caractérisée en ce que** la composition est une crème.

4. Composition selon l'une des revendications 1 à 3 **caractérisée en ce que** les agents colorants sont choisis parmi des pigments et des laques pris seuls ou en mélange.

5. Composition selon l'une des revendications 1 à 4 **caractérisée en ce que** les agents colorants sont choisis parmi les mélanges suivant :
a) FD&C blue n°2 aluminium lake + oxyde de fer jaune + dioxyde de titane
b) Hydroxyde de chrome + Oxyde de fer jaune + dioxyde de titane
c) FD&C blue n°1 aluminium lake + oxyde de fer jaune + dioxyde de titane
d) Hydroxyde de chrome + dioxyde de titane.

6. Composition selon l'une des revendications 1 à 5 **caractérisée en ce que** la quantité totale d'agents colorants n'excède pas 1% en poids de la composition.

7. Composition selon l'une des revendications 1 à 6 **caractérisée en ce qu'**elle comprend :
| | |
|---|---|
| a) | Eau purifiée |
| b) | Glycérine |
| c) | Sorbitol |
| d) | Métronidazole |
| e) | palmitate d'isopropyle |
| f) | Cire autoémulsionnable |
| g) | oxyde de fer jaune |
| h) | colorant bleu : FD&C Blue Aluminium Lake |
| i) | dioxyde de Titane |
| j) | Alcool benzylique |
| k) | Acide lactique 90% QSP PH 5 |

8. Utilisation de la composition selon l'une des revendications précédentes pour la fabrication d'un médicament destiné au traitement des affections dermatologiques, telles la rosacée, l'acné vulgaire, la dermite séborrhéique, la dermatite périorale, les éruptions acnéiformes, la dermatite acantholytique transitoire, et l'acné miliaris necrotica.

9. Utilisation de la composition selon la revendication 9 **caractérisée en ce que** l'affection dermatologique est la rosacée.

10. Mode opératoire de fabrication de la composition selon l'une des revendications 1 à 8
**caractérisé en ce qu'**il consiste à :
a) incorporer les agents colorants dans la phase huileuse par dispersion sous forte agitation, préférentiellement à l'ultra-turrax, preferentiellement à l'ultra-turrax à une vitesse de 8000 tr/min ;
b) ajouter la phase huileuse contenant les agents colorants à la phase aqueuse;
c) émulsionner les phases 10 minutes sous forte agitation ;
d) diminuer la vitesse d'agitation afin d'obtenir la viscosité recherchée pour une formule donnée.
